# EUROPEAN PATENT APPLICATION

(11) **EP 4 487 910 A1**
(43) Date of publication of application: **08.01.2025**
(21) Application number: 23460026.0
(22) Date of filing: 06.07.2023
(51) Int. Cl.: A61P 3/10, C07H 7/04, C07H 15/04, A61K 31/7034

(54) **PROCESS FOR THE PREPARATION OF DAPAGLIFLOZIN**

(71) Applicant: Zaklady Farmaceutyczne Polpharma S.A., 83-200 Starogard Gdanski (PL)
(72) Inventor: Kaminska, Ewelina, 80-288 Gdansk (PL); Punda, Pawel, 83-200 Okole (PL)
(74) Representative: Kondrat, Mariusz

(57) **Abstract**

The present invention relates to a process for the preparation of dapagliflozin, comprising the reaction of 4-(5-bromo-2-chlorobenzyl)phenyl ethyl ether starting material with an alkyl-lithium compound (step a); subsequent reaction of the obtained product with a trimethylsilyl-protected gluconolactone (step b); subsequent reaction of the obtained product with an acid in methanol to obtain the methyl C-aryl glycoside (V) (step c); and a last step, wherein the obtained product is converted to dapagliflozin, or a pharmaceutically acceptable salt or solvate thereof (step d). The selection of specific ratios and/or amounts of solvents in steps a) and c) provides an improved process, with improved overall purity with less purification steps.

## Description

### Technical Field

The present invention relates to a process for the preparation of the SGLT2 inhibitor dapagliflozin.

### Technical Background

Dapagliflozin is a reversible and highly selective inhibitor of sodium-dependent glucose cotransporter 2 (SGLT2). It is used in the treatment of type 2 diabetes mellitus, and it is also indicated for the treatment of heart failure and chronic kidney disease.

Due to the high hygroscopicity of dapagliflozin in free form, the solid form of dapagliflozin currently used in the marketed tablets is the propanediol monohydrate, which is a solvate that consists of dapagliflozin compounded with (S)-(+)-1,2-propanediol and water in a 1:1:1 ratio.

The preparation of dapagliflozin was first disclosed in the international patent application WO03/099836-A1. The synthetic route disclosed therein remains the most widely used for the preparation of dapagliflozin (Wang et al., Org. Process Res. Dev., doi: 10.1021/acs.oprd.2c00389) and it is summarized in Scheme I:

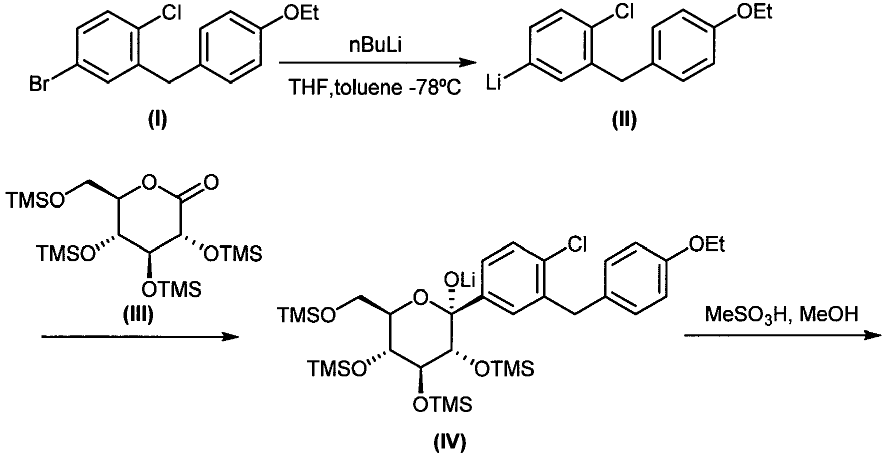

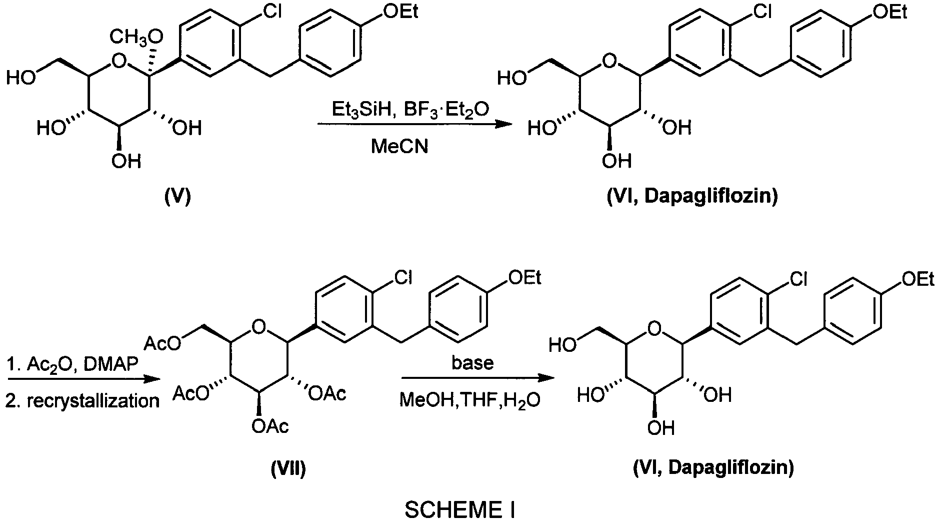

According to this method, aryl bromide (I) undergoes sequential Br/Li exchange with n-butyllithium (n-BuLi) at -78 °C, C-arylation with trimethylsilyl (TMS)-protected gluconolactone (III), and methoxylation with a methanol solution of methanesulfonic acid to obtain methyl C-aryl glycoside (V). Next, triethylsilane (Et₃SiH) and BF₃·Et₂O are used to reduce the compound of formula (V) to dapagliflozin (VI). The reduction reaction generally produces impurities, which are difficult to remove, so it is usually necessary to further improve the purity of dapagliflozin by acetylation and deacetylation, as the acetylated product (VII) is easier to crystallize.

The base originally used in the last deacetylation step was LiOH, while this strong base has been substituted in subsequent prior art documents with NaOH, for example, as disclosed in WO2008/002824-A1. Additionally, for example, other alternative acids have been disclosed to substitute the methanesulfonic acid used in the methoxylation step.

Despite the improvements described so far in the prior art, the above depicted multistep process involves the formation of different impurities along the process and, consequently, requires tedious purification procedures and results in poor yield.

For large-scale chemical syntheses, to achieve an optimisation in the purification requirements is particularly useful, as it would allow to spare resources and processing time, which would result in significant cost reduction.

However, practical achievement of such optimisation is not straightforward.

Therefore, there is the need to provide an improved method for the preparation of dapagliflozin that can afford this substance in good yield and with low levels of impurities, and reducing the need of purification steps along the process .

### Object of the invention

The object of the present invention is a process for the preparation of dapagliflozin.

### Detailed description of the invention

The object of the present invention is a process for the preparation of dapagliflozin of formula (VI): 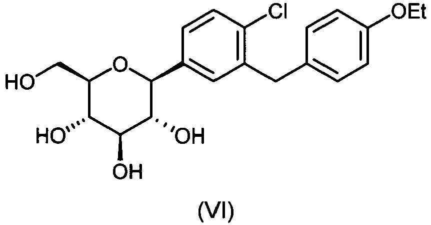 or a pharmaceutically acceptable salt or solvate thereof, comprising the following steps:
a) reacting a compound of formula (I): 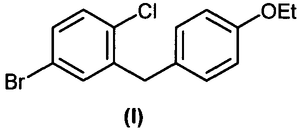 with an alkyl-lithium compound, in a mixture of solvents comprising solvent A and solvent B, to provide a compound of formula (II): 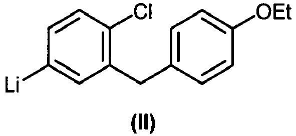
b) reacting the compound of formula (II) with a compound of formula (III): 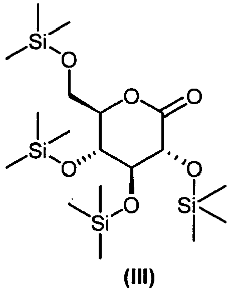 to provide a compound of formula (IV) 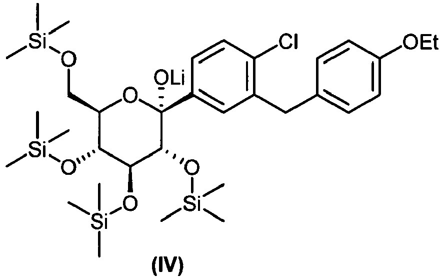
c) treating the compound of formula (IV) with an acid, in methanol, to provide a compound of formula (V) 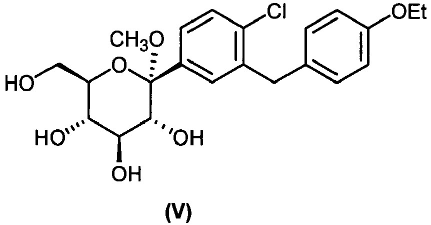 and
d) converting the compound of formula (V) to dapagliflozin, or a pharmaceutically acceptable salt or solvate thereof.
wherein in step a) the solvent A is selected from tetrahydrofuran and 2-methyltetrahydrofuran, solvent B is toluene, and the volume ratio A:B is from 1:4 to 1:6, and wherein the amount of methanol in step c) is of from 8 to 18 litres per Kg of starting compound of formula (I).

The authors of the present invention have surprisingly found that by modifying the ratio of the solvents used in the lithiation step a), combined with an increase in the amount of methanol used in the methoxylation step c), a significant increase in the purity of dapagliflozin intermediate (V) is achieved.

Without wishing to be bound to any theory, it is believed that, by these changes in the proportion of solvents, a change of polarity is achieved which allows a more efficient work-up, so that the formed compound of formula (V) and the impurities remain in different phases, and can be efficiently separated by simple phase separation.

Along the present description, as well as in the claims, the singular expressions, generally preceded by the articles "a", "an" or "the", are meant to include also the plural forms, unless the context clearly indicates otherwise. Furthermore, numeric values preceded by the term "about" are meant to include the exact stated value and also a certain variation around such value, namely a variation or ±5% of the stated amount. Numeric ranges defined by lower and upper endpoints are meant to include also said stated endpoints. The temperatures stated along the present description, relative to the different steps of the process, are meant to be the internal temperature inside the reactor. The percentages disclosed (%, wt%) are always weight percentages, unless otherwise specified.

### Step a): Lithiation

The first step of the process comprises the reaction of a compound of formula (I) with an alkyl-lithium compound, to provide a compound of formula (II):

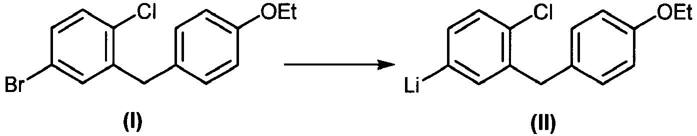

The starting compound of formula (I) (5-bromo-2-chloro-4'-ethoxydiphenylmethane, CAS 461432-23-5) is commercially available, and can also be prepared from 5-bromo-2-chlorobenzoic acid, as disclosed in WO03/099836-A1.

The alkyl-lithium compound is, for example, methyl lithium, n-butyl lithium or t-butyl lithium, among others. Preferably, the alkyl-lithium compound is n-butyl lithium.

The reaction of compound of formula (I) with the alkyl-lithium compound takes place in a mixture of solvents comprising solvent A and solvent B, wherein solvent A is selected from tetrahydrofuran (THF) or 2-methyltetrahydrofuran (2-MeTHF) and solvent B is toluene, and wherein the volume ratio A:B is from 1:4 to 1:6, preferably is about 1:5.

It is noticeable that, in the prior art, the amount of THF/2-MeTHF used, relative to toluene, is always higher. For example, in WO03/099836-A1 (page 38) the proportion THF:toluene used is 1:2. In the Indian patent application IN202141020826 (Example 1, page 14) the proportion THF:toluene is also 1:2. In EP4023644-A1 (Example 1, page 7), where the same process is used for the preparation of the analogous drug empagliflozin, the proportion 2-MeTHF:toluene is 1:3.

In one embodiment, solvent A is 2-methyltetrahydrofuran.

Concerning the amount of the mixture of solvents A+B used, it is generally from about 5 to about 14 litres per Kg of compound of formula (I), preferably from about 6 to about 13, still more preferably from about 7 to about 12, and still more preferably from about 8 to about 10 litres per Kg of compound of formula (I).

The mixture of solvents used in this step, besides A and B, may comprise an additional solvent, typically, an alkane such as, for example n-pentane, n-hexane, n-heptane, n-octane or cyclohexane; or ethers such as, for example, diethyl ether, dibutyl ether or methyl tert-butyl ether.

Typically, the compound of formula (I) is dissolved in the mixture of solvents A+B, as discussed above, while the alkyl-lithium compound is added, dissolved in said additional solvent.

The additional solvent, if present, is preferably selected from n-pentane, n-hexane, n-heptane, n-octane and cyclohexane, more preferably is n-hexane, and is typically added in an amount which ranges from about 0.5 to about 1.5 times the volume of solvent A, preferably from about 0.7 to about 1.2 times the volume of solvent A, and more preferably is about the same volume of solvent A.

The amount of the alkyl-lithium compound used in the reaction is about equimolar relative to the amount of the compound of formula (I).

This reaction typically takes place at ultralow temperature, preferably at a temperature under -50 °C, more preferably comprised in the range from about -55 °C to about -80 °C.

The obtained product (II) generally is not isolated, but it is typically used in the next step as a solution, as directly obtained in the above stated reaction conditions.

### Step b): C-arylation with trimethylsilyl-protected gluconolactone (III)

The second step of the process involves the reaction of the compound of formula (II), obtained in the previous step, with a trimethylsilyl-protected gluconolactone of formula (III) to provide the compound of formula (IV).

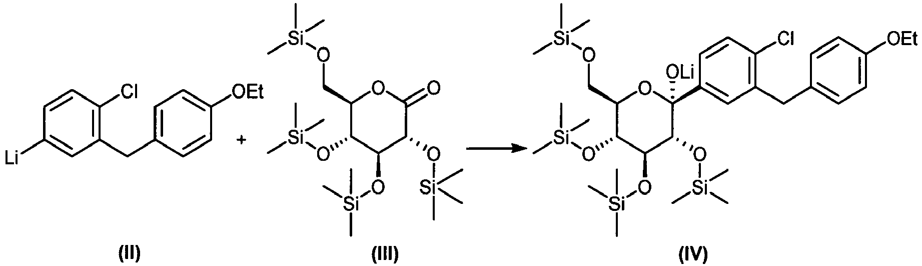

The compound of formula (III) ((3R,4S,5R,6R)-3,4,5-tris((trimethylsilyl)oxy)-6-(((trimethylsilyl)oxy)methyl)tetrahydro-2H-pyran-2-one, CAS 32384-65-9) is commercially available, or can be prepared from the corresponding gluconolactone (CAS 90-80-2) and trimethylsilyl chloride, as disclosed in WO03/099836-A1.

Compound of formula (III) is typically added dissolved in toluene, wherein the amount of toluene used is typically from 0.5 to 2, preferably from 0.6 to 1.5, more preferably from 0.7 to 1.3, still more preferably from 0.8 to 1 times the volume of toluene (solvent B) used in step a).

The temperature inside the reactor in this step is typically kept below -20 °C, preferably below -40 °C, and more preferably comprised in the range from about -55 °C to about -80 °C.

### Step c): methoxylation

In the third step of the process, the compound of formula (IV) obtained in the previous step is treated with an acid, in methanol, to provide the compound of formula (V) ((2S,3R,4S,5S,6R)-2-(4-chloro-3-(4-ethoxybenzyl)phenyl)-6-(hydroxymethyl)-2-methoxytetrahydro-2H-pyran-3,4,5-triol):

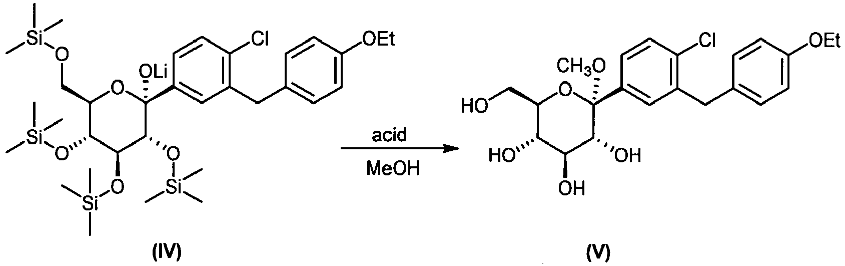

Suitably, compound of formula (IV) is used in solution, as obtained in the previous step, without isolation. To said solution, typically, a methanolic solution of the acid is added. This methanolic solution can be, for example, methanol saturated in the acid or prepared by mixing methanol with concentrated aqueous solution of the acid.

The acid is preferably selected from hydrochloric acid, sulphuric acid, methanesulphonic acid, phosphoric acid, hydrofluoric acid, hydrobromic acid and nitric acid. Preferably, the acid is hydrochloric acid.

The volume of methanol used in this step is calculated to be in the range of from 8 to 18 litres of methanol per Kg of starting compound of formula (I). Preferably, the volume of methanol is from 9 to 16, more preferably from 10 to 14 and still more preferably from 11 to 13 litres of methanol per Kg of starting compound of formula (I).

It is again noticeable that in the prior art the amount of methanol used in this step is always considerably lower. For example, in WO03/099836-A1 (page 38) the proportion used is 6.7 litres per Kg of starting compound (I). In the Indian patent application IN202141020826 (Example 1, page 14), the amount used is 5 litres per Kg of starting compound of formula (I). In EP4023644-A1 (Example 1, page 7), where the same process is used for the preparation of the analogous drug empagliflozin, the amount used is 6.5 litres per Kg of starting compound.

During the addition of the methanolic solution of the acid, the temperature is preferably kept low, typically in the range from about -80 °C to about 0 °C, preferably from about -75 °C to about -45 °C.

After the addition, the temperature may be raised, for example, to from about 10 °C to about 25 °C.

After a suitable reaction time, for example, from 1 to 10 hours, the reaction is typically quenched with an aqueous solution of a base, for example, of sodium carbonate, until a pH typically comprised between 8 and 10, preferably between 8.5 and 9.5.

Typically, after phase separation, the aqueous phase, comprising the compound of formula (V), is collected and the organic phase is discarded.

The aqueous phase is typically subjected to regular workup, using, for example, organic solvents such as ethyl acetate, n-propyl acetate, n-butyl acetate, or dichloromethane, among others, so that the product (V) can be finally obtained from the organic phase, after evaporation.

### Step d): preparation of dapagliflozin from intermediate (V)

The intermediate of formula (V) can be converted into dapagliflozin, or a pharmaceutically acceptable salt or solvate thereof, using known methods:

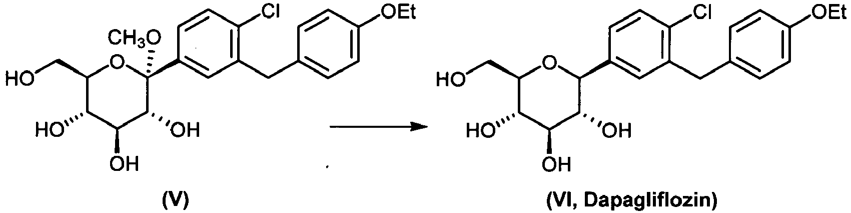

One suitable method comprises treating the compound of formula (V) with a reducing agent, for example, an organosilane (e.g. triethylsilane) in boron trifluoride diethyl etherate, in a suitable solvent (e.g. acetonitrile), as disclosed, for example, in WO03/099836-A1. Other suitable solvents are, for example, dimethyl formamide, n-hexane, dioxane or tetrahydrofuran, among others, or mixtures thereof.

After the reduction reaction, typically, the pH is adjusted to a value in the range of from about 7.0 to about 8.0, using an aqueous solution of an inorganic base, for example, sodium hydroxide, potassium hydroxide, sodium carbonate, sodium bicarbonate, potassium carbonate, potassium bicarbonate, among others. For example, aqueous sodium bicarbonate can be used. After phase separation, dapagliflozin (VI) can be obtained from the organic phase.

For improving the purity of dapagliflozin (VI), it is typically acetylated, to the acetylated product of formula (VII), which can be conveniently purified by crystallization:

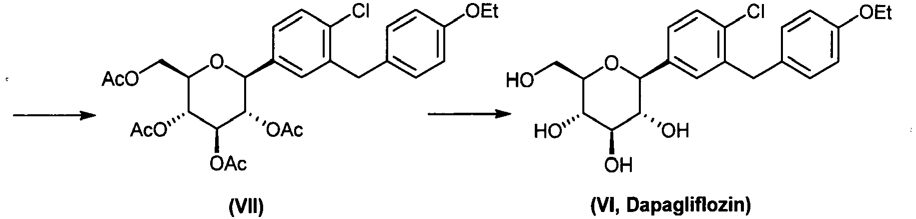

The acetylation can be also performed according to known process disclosed in the prior art. For example, by treating the obtained crude dapagliflozin with acetic anhydride and 4-dimethylaminopyridine (DMAP), as disclosed, for example, in WO03/099836-A1.

In an embodiment, the acetylation reaction is performed by using DMAP, in a suitable solvent, for example, ethyl acetate, dichloromethane or toluene. An additional base is typically added, for example, N-methylmorpholine, triethylamine or N,N-diisopropylethylamine, among others. After reaction completion, and aqueous washing workup, the dapagliflozin acetate (VII) can be obtained. It is then typically recrystallized from ethanol.

The product of formula (VII) is finally deacetylated, to provide dapagliflozin, by treatment with a suitable base, for example, with an aqueous solution of an inorganic base, such as sodium hydroxide, potassium hydroxide or lithium hydroxide, preferably sodium hydroxide or potassium hydroxide. The acetylated product of formula (VII) is typically dissolved in a mixture of solvents comprising, for example, tetrahydrofuran and an aliphatic alcohol, such as methanol or ethanol, and the aqueous solution of the base, typically, NaOH aqueous solution, is added.

Dapagliflozin can be obtained from the reaction mixture, using conventional workup. For example, by extraction with a suitable organic solvent (e.g. ethyl acetate or methyl *tert-*butyl ether), followed by successive washings with water or brine, and distillation. The residue can be optionally again redissolved in an organic solvent and again washed and distilled.

Dapagliflozin is obtained as a residue after distillation.

Dapagliflozin can be then recovered in solid form, for example, as amorphous dapagliflozin. To this end, the residue is redissolved in a suitable organic solvent or organic solvent mixture, for example, *tert*-butyl methyl ether, methanol, ethanol, dichloromethane, acetonitrile, tetrahydrofuran, 2-methyltetrahydrofuran, among others, or mixtures thereof; and an antisolvent is subsequently added, for example, an n-alkane, such as n-heptane or n-hexane, or cyclohexane, among others.

Alternatively, dapagliflozin can be recovered in solid form as a solvate, in particular, as dapagliflozin propanediol monohydrate. To this end, the residue is redissolved in a solvent or a mixture of solvents, typically selected from ethyl acetate, isopropyl acetate, n-propyl acetate, butyl acetate, n-pentane, n-hexane, n-heptane, n-octane, cyclohexane, among others, or mixtures thereof. Water and (*S*)-(+)-1,2-propanediol are added, and crystalline dapagliflozin propanediol monohydrate is obtained from the solution, typically after seeding.

### Examples

### Example 1 Preparation of dapagliflozin according to the process of the invention

### Step a: Preparation of 4-chloro-3-(4-ethoxybenzyl)phenyl)lithium (II)

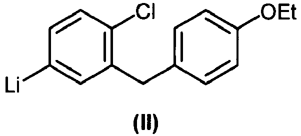

4-(5-bromo-2-chlorobenzyl)phenyl ethyl ether (30.6 g) was dissolved in 2-methyltetrahydrofuran (45 mL) and toluene (225 mL) at ambient temperature. The solution was cooled down to temperature below -70°C under flow of inert gas and subsequently 2.5M solution of butyllithium in hexanes (41.5 mL) was added dropwise maintaining temperature in the range of -75°C to -55°C, to provide a solution of (II).

### Step b: Preparation of lithium (3R,4S,5R,6R)-2-[4-chloro-3-(4-ethoxybenzyl)phenyl]-3,4,5-tris(trimethylsilyl)oxy]-6-{[(trimethylsilyl)oxy]methyl} tetrahydro-2H-pyran-2-olate (IV)

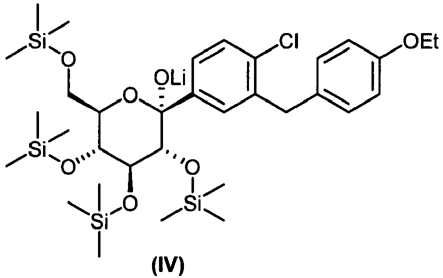

Simultaneously (3R,4S,5R,6R)-3,4,5-tris[(trimethylsilyl)oxy]-6-{[(trimethylsilyl)oxy] methyl}tetrahydro-2H-pyran-2-one (III, 43.87 g) was dissolved in toluene (182 mL) at ambient temperature. The solution was cooled down to temperature below -70°C under flow of inert gas. Next the cold solution of (II) (T<-55°C) was added dropwise to solution of (III), to obtain a solution of the title compound.

### Step c: Preparation of methyl 1-C-[4-chloro-3-(4-ethoxybenzyl)phenyl]-α-D-glucopyranoside (V)

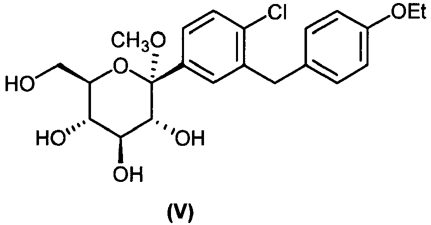

To cold solution of (IV) (T<-55°C) a methanolic solution of concentrated aqueous HCl (prepared with 332 mL of MeOH and 17 mL of 34% HClaq) was added dropwise. Next the temperature was raised to RT (T=20°C), which was maintained for 3h, and then the reactor content was cooled down to about 0°C.

Aqueous 5% Na₂CO₃ (about 150 mL) was added dropwise, keeping the temperature below 10°C, to pH=8.5-9.5. Phases were separated and aqueous layer was collected with compound of formula (V).

To the aqueous layer Brine 20% (150 mL) was added and aqueous solution was extracted triple time with EtOAc (150 mL). Combined organic layers were washed with 20% brine (2x 100 mL). Organic phase was concentrated in vacuo (p=230-30mbar; T=50°C). Next, toluene (100 mL) was added into residue after distillation and the mixture was evaporated under vacuo. This operation was repeated again with toluene (100 mL) and the residue after distillation was held under vacuo for 30 min at p=30mbar. The compound of formula (V) was obtained as an oily residue (35.1 g).

### Step d1: Preparation of crude dapagliflozin

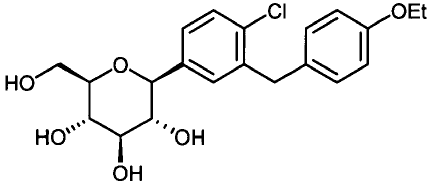

Into reactor with compound of formula (V) acetonitrile (195 mL) was added. Stirring to dissolution at 20-35°C. Into second reactor acetonitrile (117 mL) and triethylsilane (21.7 g) were added, and the content was cooled down to the temperature of about -15°C under flow of inert gas with stirring. Boron trifluoride diethyl etherate (39.8 g; ≥46.5% BF₃ basis) was added fast dropwise. Solution of compound of formula (V) was added dropwise to the second reactor. Temperature was raised to about 5°C, stirring for about 1h. 10% NazCOs aqueous solution (200 mL) was added dropwise at a temperature of 5-12°C. After phase separation (pH of water phase was about 7) organic phase was washed with 20% brine and was concentrated in vacuo (p=300-150mbar; T=50°C). Ethyl acetate (80 mL) and toluene (155 mL) were added followed by concentration under reduced pressure (p=120-30mbar; T=50°C), to provide the title compound.

### Step d2: Preparation of (1S)-2,3,4,6-tetra-O-acetyl-1,5-anhydro-1-[4-chloro-3-(4-ethoxybenzyl)phenyl]-D-glucitol (VII)

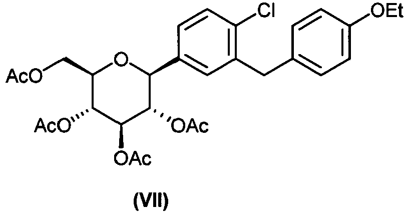

To crude dapagliflozin obtained in previous step, 4-dimethylaminopyridine (0.57 g) and ethyl acetate (390 mL) were added followed by dissolution with stirring under inert gas atmosphere at RT. Next, N-methylmorpholine (52 mL) was added. Acetic anhydride (45 mL) was added dropwise keeping temperature below 30°C, and stirring for 1-2h. The mixture was cooled down to about 0°C and drinking water (313 mL) was added in one portion. The temperature was raised to RT, phases were separated, and organic phase was washed with: 1M HCl aqueous solution, Na₂CO₃ aqueous solution, and 20% brine. Organic phase was concentrated in vacuum (p=230-30mbar; T=50°C), to provide the title compound.

Compound of formula (VII) (residual after distillation) was dissolved in ethanol denaturated with acetone (314 mL) with stirring and warming up to boiling point. Next, the solution was cooled down to about 65°C, seeded with 0.2 g of compound of formula (VII), maintained at about 65°C for 1h and cooled to 15-25°C during 5 hours. After stirring at 15-25°C for 3h, solids were filtered and washed with solvent (40 mL; 99.8% EtOH:acetone 100:3.5 V/V) and dried at about 50°C. 35.15 g of compound of formula (VII) were obtained (65% yield).

### Step d3: Preparation of purified dapagliflozin

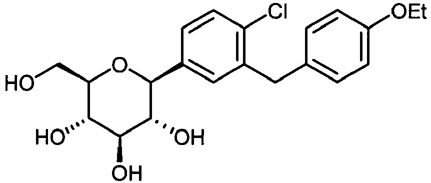

The compound of formula (VII) (16.1 g) was dissolved in tetrahydrofuran (105 mL) with stirring under inert gas atmosphere at room temperature. Methanol was added (48.5 mL). Next, solution of sodium hydroxide (4.5 g) in water (43.5 mL) was added dropwise keeping internal temperature below 30°C. After 1h under stirring, additional amount of solid NaOH can be added if reaction is not completed (0.9 g). Activated charcoal (1.6 g) was added followed by stirring 30-60 min. After filtration methyl tert-butyl ether (177 mL) and 20% brine (95 mL) were added. After phase separation organic phase was washed twice with 20% brine. Addition of toluene (65 mL) was followed by distillation under reduced pressure (p≤200mbar; T=50°C).

To the oily residue obtained, methyl tert-butyl ether (33 mL) was added to dissolution. Organic phase was washed twice with water DEMI at temperature range of 40-50°C. The solution was concentrated in vacuo (p=400÷100mbar; T=50°C) and the residue was dissolved in methyl tert-butyl ether (105 mL) followed by toluene (65 mL) addition and reduced pressure distillation (p=400+30mbar; T=50°C), to provide white foamy solid of pure dapagliflozin.

The residue after distillation was dissolved in methyl tert-butyl ether (247 mL) at a temperature in the range 40-50C°. After cooling to T=20-25°C, the solution was added dropwise to n-heptane (516 mL) at a temperature in the range of 20-25°C. The obtained suspension was stirred for about 3h. Solids were filtered and washed with n-heptane (81 mL). Wet dapagliflozin was dried in vacuum drier at a temperature in the range 20-40°C for two days.

9.0 g of dapagliflozin were obtained (79% yield), purity 99.78% by UHPLC. Dapagliflozin was obtained as amorphous dapagliflozin. The overall yield of the process was 51.4%.
¹H NMR (500 MHz. DMSO-d6) δ 7.35 (d, J = 8.4 Hz, 1H), 7.30 (d, J = 1.6 Hz, 1H), 7.21 (dd, J = 8.4, J = 1.6 Hz, 1H), 7.08 (d, J = 8.5 Hz, 2H), 6.81 (d, J = 8.5 Hz, 2H), 3.96 (1H), 3.94 (m, 2H), 3.42-3.67 (m, 1H), 3.24 (t, J = 8.8 Hz, 1H), 3.20 (m, 1H),3.14 (t, J = 8.8 Hz, 1H), 3.09 (t, J = 8.8 Hz, 1H), 1.28 (t, 3H).
¹³C NMR (125 MHz. DMSO-d6) δ 156.9, 139.7, 137.8, 131.9, 131.2, 130.8, 129.6, 128.7, 127.4, 114.3, 81.2, 80.7, 78.3, 74.7, 70.3, 62.9, 61.3, 37.7, 14.7

### Comparative Example: Preparation of dapagliflozin according to the prior art

Steps a), b) and c) were repeated as disclosed in Example 1, but using the conditions reported in the prior art. The conditions were approximately as reported in EP4023644-A1 (Example 1).

Therefore, in step a) 4-(5-bromo-2-chlorobenzyl)phenyl ethyl ether (30.6 g) was dissolved in 2-methyltetrahydrofuran (68 mL) and toluene (203 mL) at ambient temperature. The solution was cooled down to temperature below -70°C under flow of inert gas and subsequently 2.5M solution of butyllithium in hexanes (41.5 mL) was added dropwise maintaining temperature in the range -75°C to -55°C, to provide a solution of compound of formula (II).

Step b) was performed in the same way as disclosed above, to obtain a solution of compound of formula (IV).

In step c), to the cold solution of compound of formula (IV) (T<-55°C) obtained, methanol (120 mL) was added dropwise. Then methanolic solution of concentrated aqueous HCl (prepared with 68 ml of MeOH ml and 12 mL of 34% HCl aq) was added dropwise. Next the temperature was raised to RT, maintained for 3h and then cooled down to about 0°C.

Aqueous 4% Na₂CO₃ was added dropwise keeping temperature below 10°C to pH=8.5-9.5 (about 125 mL). Phases were separated and aqueous layer was collected with compound of formula (V).

To the aqueous layer Brine 20% (150 mL) was added and aqueous solution was extracted triple time with EtOAc (150 mL). Combined organic layers were washed with 20% brine (2x 100 mL). Organic phase was concentrated in vacuo (p=230-30mbar; T=50°C). Next, toluene (100 mL) was added to the residue after distillation and the mixture was evaporated under vacuo. This operation was repeated again with toluene (100 mL) and the residue after distillation was held under vacuo for 30 min at p=30mbar. Compound of formula (V) was obtained as oily residue after distillation (13.04 g).

The following table shows the concentration of compound of formula (V) in the aqueous layer and in the organic layer after quenching with sodium carbonate and phase separation, as well as the amount of compound of formula (V) obtained as oily residue from the organic phase after workup:

| | Example 1 | Comparative Example |
|---|---|---|
| *Concentration of compound (V) in the phases after quenching with sodium carbonate* | | |
| Aqueous layer | 84.6 mg/ml | 42 mg/ml |
| Organic layer | 6.2 mg/ml | 35 mg/ml |

| *Amount of compound (V) obtained after workup and distillation of organic phase* | | |
|---|---|---|
| | 35.1 g | 13.04 g |

With the process of the invention, intermediate compound of formula (V) goes mainly to the aqueous phase, allowing an easy and effective separation of the impurities. This fact allows for obtaining this intermediate with higher purity, without the need for burdensome purification steps.

This allows for the preparation of dapagliflozin with overall higher yield, with fewer steps due to reduced purification operations, so allowing a reduction of production costs.

## Claims

1. A process for the preparation of dapagliflozin of formula (VI): 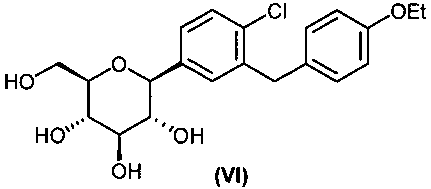 or a pharmaceutically acceptable salt or solvate thereof, comprising the following steps:
a) reacting a compound of formula (I): 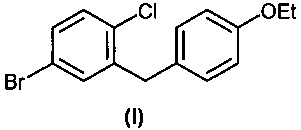 with an alkyl-lithium compound, in a mixture of solvents comprising solvent A and solvent B, to provide a compound of formula (II): 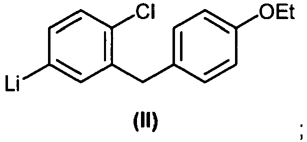
b) reacting the compound of formula (II) with a compound of formula (III): 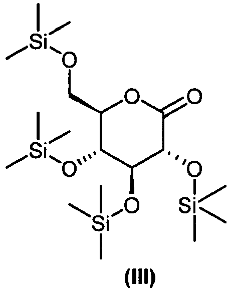 to provide a compound of formula (IV) 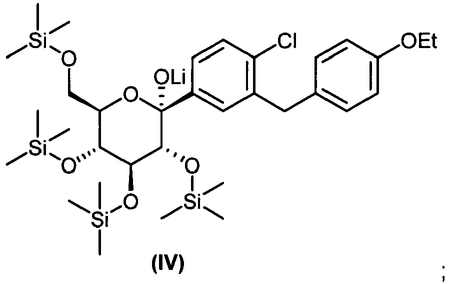
c) treating the compound of formula (IV) with an acid, in methanol, to provide a compound of formula (V) 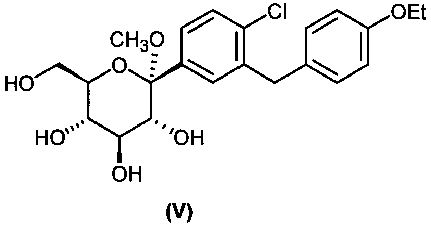 and
d) converting the compound of formula (V) to dapagliflozin, or a pharmaceutically acceptable salt or solvate thereof;
wherein in step a) the solvent A is selected from tetrahydrofuran and 2-methyltetrahydrofuran, solvent B is toluene, and the volume ratio A:B is from 1:4 to 1:6, and wherein the amount of methanol in step c) is of from 8 to 18 litres per Kg of starting compound of formula (I).

2. Process according to claim 1, wherein the alkyl-lithium compound in step a) is selected from methyl lithium, n-butyl lithium and t-butyl lithium, and preferably is n-butyl lithium.

3. Process according to claims 1 or 2, wherein solvent A in step a) is 2-methyltetrahydrofuran.

4. Process according to any one of claims 1 to 3, wherein the amount of the mixture of solvents A+B used in step a), is from 5 to 14 litres per Kg of compound of formula (I).

5. Process according to any one of claims 1 to 4, wherein the acid in step c) is selected from hydrochloric acid, sulphuric acid, methanesulfonic acid, phosphoric acid, hydrobromic acid and nitric acid.

6. Process according to claim 5, wherein the acid is hydrochloric acid.

7. Process according to any one of claims 1 to 6, wherein the amount of methanol in step c) is from 9 to 16 litres, preferably from 10 to 14 litres, and more preferably from 11 to 13 litres per Kg of compound of formula (I).

8. Process according to any one of claims 1 to 7, wherein step d) comprises the following steps:
d1) converting the compound of formula (V) to dapagliflozin,
d2) converting dapagliflozin to dapagliflozin acetate of formula (VII): 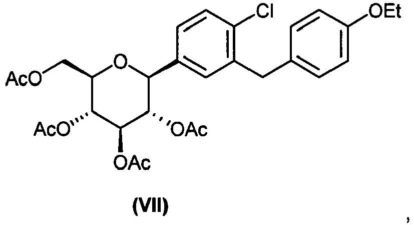 and
d3) converting dapagliflozin acetate of formula (VII) to dapagliflozin or a pharmaceutically acceptable salt or solvate thereof.

9. Process according to claim 8, wherein, before step d3), the compound of formula (VII) is recrystallized from ethanol.

10. Process according to claims 8 or 9, wherein in step d3) dapagliflozin is obtained as amorphous dapagliflozin.

11. Process according to claims 8 or 9 , wherein in step d3) dapagliflozin obtained as crystalline dapagliflozin propanediol monohydrate.
